Europäisches Patentamt

European Patent Office

Office européen des brevets

(11) Numéro de publication: **0 307 300**
**A1**

## (12) DEMANDE DE BREVET EUROPEEN

(21) Numéro de dépôt: 88402233.6

(22) Date de dépôt: 06.09.88

(51) Int. Cl.4: **G 10 K 11/34**

(30) Priorité: 07.09.87 FR 8712385

(43) Date de publication de la demande:
15.03.89 Bulletin 89/11

(84) Etats contractants désignés:
AT BE CH DE ES FR GB GR IT LI LU NL SE

(71) Demandeur: TECHNOMED INTERNATIONAL S.A.
28, rue Desaix
F-75015 Paris (FR)

INSTITUT NATIONAL DE LA SANTE ET DE LA
RECHERCHE MEDICALE (INSERM)
101, rue de Tolbiac
F-75654 Paris Cédex 13 (FR)

(72) Inventeur: Cathignol, Dominique
14, Rue du Fort
F-69740 Genas (FR)

Lacruche, Bernard
29, Rue Alphonse Bordereau
F-77500 Chelles (FR)

Mestas, Jean-Louis
1, Impasse Marc Seguin
F-69680 Chassieu (FR)

(74) Mandataire: Portal, Gérard et al
Cabinet Beau de Loménie 55, rue d'Amsterdam
F-75008 Paris (FR)

(54) Dispositif piézoélectrique à ondes négatives réduites, et utilisation de ce dispositif pour la lithotritie extra corporelle ou pour la destruction de tissus particuliers.

(57) L'invention concerne un dispositif générateur d'ondes ultrasoniques focalisées en un foyer.

Ce dispositif générateur comprend une pluralité d'éléments transducteurs (2, 4, 6) disposés extérieurement sur un moyen support de focalisation en un point de focalisation (10) ou foyer, par exemple une coupole sphérique, et est caractérisé en ce que les éléments transducteurs (2, 4, 6) sont répartis en au moins deux groupes d'éléments transducteurs, dont la fréquence de résonance differe de telle sorte que les ondes ultrasoniques positives principales émises par chaque groupe s'ajoutent et les ondes ultrasoniques secondaires positives et négatives s'annulent au moins partiellement.

Ainsi, on réduit de manière significative les ondes négatives.

Ce dispositif peut être utilisé avantageusement pour la lithotritie extra corporelle.

Fig.2

EP 0 307 300 A1

## Description

### Dispositif piézoélectrique à ondes négatives réduites, et utilisation de ce dispositif pour la lithotritie extra corporelle ou pour la destruction de tissus particuliers

La présente invention concerne essentiellement un dispositif piézoélectrique à ondes négatives réduites, et l'utilisation de ce dispositif pour la lithotritie extra corporelle ou pour la destruction de tissus particuliers.

On sait qu'un générateur piézoélectrique est essentiellement composé d'un, de plusieurs ou d'un très grand nombre d'éléments piézoélectriques disposés de préférence sur une coupole sphérique permettant de focaliser l'ensemble des ondelettes créées par chaque particule élémentaire constituant le ou les transducteurs (voir par exemple le document Ultrasonics, vol. 5, d'Avril 1967, pages 105-112 ; P.P. LELE).

On a également décrit un dispositif générateur d'ondes ultrasoniques focalisées en un foyer, comprenant une pluralité d'éléments transducteurs de type piézoélectrique disposés extérieurement sur une coupole sphérique, ainsi que son utilisation dans un but thérapeutique, par exemple pour la production de lésions oculaires contrôlées (voir COLEMAN, American Journal of Ophtalmology 86:185-192, 1978). COLEMAN décrit en outre la présence paraxiale d'un transducteur de diagnostic.

L'expérience montre que la qualité de la focalisation ou en d'autres termes le volume de la tache focale sera d'autant plus petit que le coefficient de remplissage sera voisin de 1 et que l'ouverture définie par l'angle de la coupole sphérique sera élevé.

En outre, on sait également que la forme de l'onde au point focal est identique ou très voisine et de toute manière étroitement dépendante de la forme de l'onde émise au niveau du transducteur piézoélectrique. Dans le cas d'un transducteur dont une face est en contact avec l'eau et la deuxième face en contact avec l'air (montage le plus simple possible et le plus courant), l'onde émise, suite à une impulsion électrique, se présente sous la forme d'une sinusoïde amortie.

Celle-ci présente ainsi des ondes positives et négatives.

Il a pu être observé que les ondes négatives sont dangereuses pour les tissus du fait qu'elles peuvent induire des effets de cavitation. (Voir article de COLEMAN et al. dans "Ultrasound in Med. & Biol. Vol. 13, No. 2, pp 69-76, 1987).

On a déjà proposé dans l'art antérieur de créer des ondes dites unipolaires (document DE-34 25 992).

Lorsque le transducteur est soumis à une impulsion très brève, il émet une série d'impulsions, la face avant émettant des ondes de polarité directe tandis que la face arrière émet des ondes de polarité inverse. L'onde résultante émise est bien entendu la somme de ces ondes qui est donc tantôt positive, tantôt négative.

La solution proposée dans le document DE-34 25 992 a pour but de "séparer" ces ondes positives et négatives. Pour cela, la face arrière est taillée de manière irrégulière de telle sorte que les ondes réfléchies par la face arrière ne soient pas ou très mal focalisées. Il en résulte ainsi que le rapport entre l'onde positive et l'onde négative croît au fur et à mesure que l'on se rapproche du point focal.

Dans cette solution, on doit noter que la deuxième onde positive sera également mal focalisée puisqu'elle est due à la réflection de la première onde sur la face arrière du transducteur.

Il est également connu d'adapter le transducteur piézoélectrique en disposant sur la face avant un matériau dont l'impédance est comprise entre l'impédance du transducteur et celle de l'eau qui sert de milieu de transmission de l'onde ultrasonique jusqu'à la cible disposée au point focal (voir le document Vol I. Part A Physical acoustics de MASON, Academic Press).

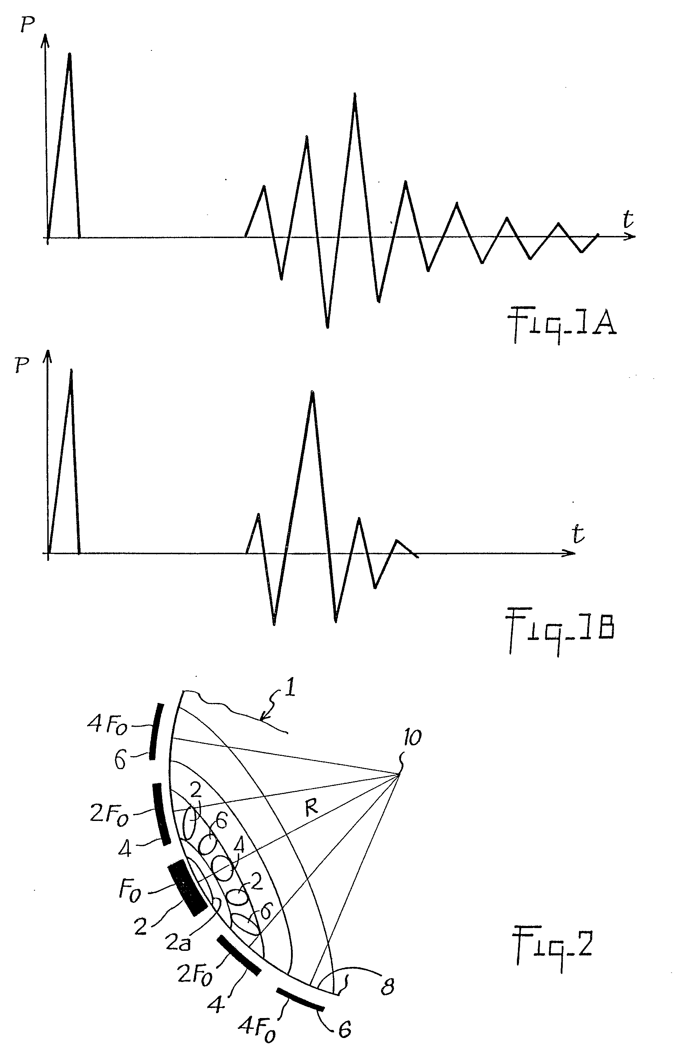

On constate alors d'une part que la durée du signal est très courte et d'autre part que la valeur maximale de la pression atteinte est beaucoup plus forte comme il ressort de la comparaison des figures 1a (ondes ultrasoniques émises non adaptées) et de la figure 1b (ondes ultrasoniques émises par un transducteur adapté).

Ainsi, dans le système unipolaire tel que préconisé par DE-34 25 992, étant donné que seule la première onde est focalisée, et que malheureusement celle-ci est toujours faible comparativement aux 2ème et 3ème ondes, le rapport de l'onde focalisée entre un transducteur adapté et un transducteur non adapté est de l'ordre d'un facteur 10, mais il et aisé de se rendre compte que dans le cas d'un transducteur non adapté et dont la face arrière n'est pas taillée irrégulièrement le système génère une onde négative d'amplitude égale à l'onde positive (voir figure 1c).

Autrement dit, malgré l'adoption de cette solution, l'onde négative est toujours présente et d'une intensité telle qu'elle est capable d'induire des effets de cavitation dangereux pour les tissus avoisinants, soit de la concrétion, soit des tissus particuliers que l'on veut détruire.

La présente invention a donc pour but de résoudre le nouveau problème technique consistant à trouver une solution réduisant ou éliminant l'amplitude de l'onde négative présente de manière inhérente dans l'onde sinusoïdale émise par tout transducteur piézoélectrique.

La présente invention a encore pour but de résoudre le nouveau problème technique consistant à trouver une solution réduisant ou éliminant non seulement l'onde négative inhérente à l'onde sinusoïdale émise par un transducteur piézoélectrique, mais encore à maintenir au moins au même niveau la hauteur de pics de l'onde émise, et donc la valeur du pic de pression comparativement aux techniques antérieurement proposées et notamment celles qui réduisent l'amplitude de l'onde négative.

Ces nouveaux problèmes techniques sont résolus pour la première fois par la présente invention de manière satisfaisante, utilisable à l'échelle industrielle.

Ainsi, la présente invention fournit un dispositif générateur d'ondes ultrasoniques focalisées en un foyer à ondes négatives réduites, comprenant une pluralité d'éléments transducteurs de type piézoélectrique disposés extérieurement sur un moyen support de focalisation en un point de focalisation ou foyer, par exemple une coupole de forme sphérique, caractérisé en ce que lesdits éléments transducteurs sont répartis en au moins deux groupes d'éléments transducteurs dont la fréquence de résonance diffère de telle sorte que les ondes ultrasoniques positives principales émises par chaque groupe s'ajoutent et les ondes ultrasoniques secondaires positives et négatives s'annulent au moins partiellement.

Selon un mode de réalisation particulièrement avantageux, le deuxième groupe d'éléments transducteurs, ou les autres groupes d'éléments transducteurs, a une fréquence de résonance voisine ou égale à un multiple ou sous multiple de la fréquence de résonance des éléments transducteurs du premier groupe.

Selon un mode de réalisation particulier, la fréquence de résonance du deuxième groupe d'éléments transducteurs est égale ou voisine du double de la fréquence de résonance du premier groupe d'éléments transducteurs.

Selon un autre mode de réalisation particulièrement avantageux, les éléments transducteurs de chaque groupe sont disposés alternativement de telle sorte que la surface totale occupée par les éléments de chaque groupe soit sensiblement identique.

Selon un autre mode de réalisation particulier de l'invention, tous les éléments transducteurs des différents groupes sont à la même distance du point de focalisation, dite distance focale. Lorsque le moyen support est une coupole sphérique, la distance focale est de préférence égale au rayon de courbure de la coupole.

Dans le cas le plus simple, où la distance focale est la même pour tous les éléments transducteurs des divers groupes, on obtient une coïncidence des pics de pression maximale entre les differents groupes qui ont des fréquences de résonance différentes par une attaque électrique de chaque groupe de fréquence donnée en envoyant des impulsions ou un front de tension retardée ou avancée en fonction du groupe attaqué par rapport au groupe de référence, l'écart de temps (retard ou avance) est donné par la formule suivante : $\Delta t = \lambda / 4\ V$

dans laquelle :

V est la vitesse ultrasonore dans le milieu (en général l'eau),

$\lambda$ est la longueur d'ondes du groupe attaqué.

Dans le cas où le moyen support est plan, on peut réaliser une focalisation électronique comme décrit dans le document DE-A-31 19 295.

Selon encore un autre mode de réalisation particulier, lorsque le moyen support est constitué par une coupole sphérique, la distance déterminée entre la face avant des éléments transducteurs d'un groupe donné et le point de focalisation est différente de la même distance au point de focalisation des autres groupes de fréquence de résonance différente, l'attaque électrique de tours les groupes est simultanée pour que les pics de pression maximale coïncident.

Par exemple, l'écart de distance focale entre le premier groupe et le deuxième groupe est de l'ordre de 1/4 de longuer d'onde. On voit que cette compensation correspond à la compensation en temps ($\Delta t$) lorsque la distance focale est constante.

En outre, lorsque la fréquence de résonance d'un groupe donné est plus faible que la fréquence de résonance du premier groupe dit de référence, la distance des transducteurs au point de focalisation de ce groupe donné est plus grande que la distance des transducteurs au point de focalisation du groupe de référence, ce qui veut dire que les éléments transducteurs de ce groupe donné sont disposés en retrait par rapport aux éléments transducteurs du groupe de référence.

De même, si la fréquence de résonance est plus élevée pour les éléments transducteurs du groupe donné, la distance des transducteurs au point de focalisation est plus petite que celle du groupe d'éléments transducteurs de référence, c'est-à-dire que les éléments transducteurs de ce groupe donné de fréquence de résonance plus élevée sont disposés en avant par rapport aux éléments transducteurs du groupe de référence.

Selon un autre mode de réalisation particulièrement avantageux, ce dispositif comprend un troisième groupe d'éléments transducteurs dont la fréquence de résonance est égale ou voisine de 4 fois la fréquence de résonance du premier groupe d'éléments transducteurs.

Comme cela est clairement compréhensible à un homme du metier, la somme des ondes émises par les groupes de transducteurs de fréquence de résonance différente va résulter en une onde résultante dont l'importance des ondes négatives sera réduite car grâce à cette combinaison, les ondes positives principales s'ajoutent tandis que les ondes négatives sont annulées au maximum, notamment par les ondes positives secondaires. On observera que la somme totale des ondes positives principales produit un pic de pression identique à celui produit dans le cas classique de l'emploi d'une seule fréquence de résonance pour la totalité des éléments transducteurs.

On obtient ainsi un effet totalement inattendu et non évident pour un homme du métier désirant détruire des concrétions (lithotrypsie) ou des tissus particuliers (opérations chirurgicales), ou même l'homme de métier de la technique ultrasonique.

D'autres buts, caractéristique et avantages de l'invention apparaîtront clairement à l'homme du métier à partir de la description explicative faite en référence aux figures annexées dans lesquelles :

- la figure 1a représente l'onde sinusoïdale émise par un élément transducteur piézoélectrique non adapté, de fréquence de résonnance $F_o$,

- la figure 1b représente le même transducteur piézoélectrique adapté par le dépôt d'une couche de résine époxyde sur la face émettrice,

- la figure 2 représente schématiquement un dispositif générateur d'ondes ultrasoniques focalisées en un foyer, à coupole de focalisation, ici sphérique, selon la présente invention,

- la figure 2a représente une vue en plan de la face interne de la coupole de focalisation selon la variante de réalisation à trois groupes d'éléments transducteurs de fréquence de résonance, objet de la figure 2,

- la figure 2b représente une vue en plan de la face interne de la coupole de focalisation selon une variante de réalisation à deux groupes d'éléments transducteurs de fréquence différente,

- la figure 3a représente individuellement l'onde sinusoïdale émise par le premier groupe de fréquence de résonnance $F_o$ et le deuxième groupe de fréquence de résonance $2F_o$, et

- la figure 3b représente l'onde résultante, résultant de la somme des deux ondes respectivement du premier groupe et du deuxième groupe.

En référence aux figures 2, 2a et 2b, on a représenté un dispositif générateur d'ondes ultrasoniques focalisées en un foyer, à ondes négatives réduites selon la présente invention.

Ce dispositif générateur représenté par la numéro de référence général 1 est du type bien connu dans la technique des générateurs ultrasoniques, qui est partiellement résumée dans la partie introductive de la présente description.

Ainsi, ce dispositif générateur 1 comprend une pluralité d'éléments transducteurs de type piézoélectrique 2, 4, 6, disposés extérieurement sur un moyen support 8 de focalisation en un point de focalisation, ou point focal 10. Ce moyen support 8 ist ici par exemple constitué par une coupole sphérique. Cette structure étant bien connue à l'homme de l'art n'est pas décrite plus en détail, ni les moyens permettent de transmettre des impulsions aux éléments transducteurs.

Selon la présente invention, ce dispositif est caractérisé en ce que les éléments transducteurs sont répartis en au moins deux groupes d'éléments transducteurs, à savoir ici par exemple un premier groupe d'éléments transducteurs 2, un deuxième groupe d'éléments transducteurs 4 et un troisième groupe d'éléments transducteurs 6, dont la fréquence de résonance diffère de telle sorte que les ondes ultrasoniques positives principales émises par chaque groupe s'ajoutent et les ondes ultrasoniques secondaires (positives et négatives) s'annulent au moins partiellement.

Selon un mode de réalisation particulièrement avantageux, le deuxième groups d'éléments transducteurs (4), ou les autres groupes d'éléments transducteurs (4, 6), présente une fréquence de résonance voisine ou égale à un multiple ou sous multiple de la fréquence de résonance des éléments transducteurs (2) du premier groupe, en particulier à un multiple de cette fréquence.

Selon un mode de réalisation particulièrement préféré, la fréquence de résonance du deuxième groupe d'éléments transducteurs 4 est égale ou voisine du double de la fréquence de résonance $F_o$ du premier groupe d'éléments transducteurs 2.

Selon un autre mode de réalisation particulier, tel que représenté, les éléments transducteurs de chaque groupe sont disposés alternativement de telle sorte que la surface totale occupée par les éléments transducteurs de chaque groupe est sensiblement identique comme cela est clairement compréhensible à partir de la considération des figures 2, 2a et 2b.

Par exemple à la figure 2a, qui représente une variante de réalisation à trois groupes d'éléments transducteurs de fréquence de résonance différente, disposés alternativement et repérés respectivement pour le premier groupe d'éléments transducteurs (2), pour le deuxième groupe d'éléments transducteurs (4) et pour le troisième groupe d'éléments transducteurs (6). Dans le cas de la présence de ce troisième groupe d'éléments transducteurs 6, la fréquence de résonance de ces éléments transducteurs 6 est égale à 4 fois ou voisine de 4 fois la fréquence de résonance $F_o$ du premier groupe.

A titre d'exemple, cette fréquence de résonance $F_o$ du premier groupe d'éléments transducteurs 2 est égale à 0,5 mégaHertz.

En outre, à la figure 2b, qui représente une autre variante de réalisation à deux groupes d'éléments transducteurs de fréquence de résonance différente, la position centrale est par exemple occupée par un élément transducteur (2) du premier groupe tandis que chaque secteur périphérique est occupé alternativement par des éléments transducteurs du premier groupe (élément 2) et des éléments transducteurs du deuxième groupe (élément 4).

Par ailleurs, pour obtenir des fréquences de résonance différentes, l'épaisseur des éléments transducteurs 2 du premier groupe est le double ou voisin du double de l'épaisseur des éléments transducteurs 4 du deuxième groupe et, dans le cas de la présence d'un troisième groupe d'éléments transducteurs 6, est égale à fois ou est voisine de 4 fois l'épaisseur des éléments transducteurs 6 du troisième groupe.

Selon un mode de réalisation le plus simple, tous les éléments transducteurs (2, 4, 6) des differents groupes sont à la même distance du point de focalisation 10, dite distance focale.

Lorsque le moyen support est une coupole sphérique, comme représenté à la figure 2, la distance focale est de préférence égale au rayon de courbure de la coupole.

Dans le cas le plus simple, où la distance focale est la même pour tous les éléments transducteurs (2, 4, 6) des divers groupes, on obtient une coïncidence des pics de pression maximale entre les differents groupes qui ont des fréquences de résonance différente par une attaque électrique de chaque groupe de fréquence donnée en envoyant des impulsions ou un front de tension retardée ou avancée en fonction du groupe attaqué par rapport au groupe de référence, l'écart de temps (retard ou avance) étant donné par la formule suivante :

$$\Delta t - \lambda / 4V$$

dans laquelle :

V est la vitesse ultrasonore dans le milieu remplissant la coupole, en général constitué par de l'eau,

$\lambda$ est la longueur d'ondes du groupe attaqué.

L'homme du métier saura ainsi clairement comment réaliser les attaques électroniques de chaque groupe de fréquence pour obtenir une coïncidence des pics de pression maximale entre les differents groupes au point de focalisation 10.

Par exemple si la fréquence de résonance $F_0$ du premier groupe d'éléments piézoélectriques 2 est de 0,5 mégaHertz et la fréquence de résonance du deuxième groupe d'éléments piézoélectriques 4 est le double de celle-ci, soit 1 mégaHertz, l'attaque électrique du deuxième groupe se fera avec un retard de l'ordre de 1/4 de période T, soit 250 nanosecondes.

Selon un autre mode de réalisation possible, notamment lorsque le moyen support est constitué par une coupole sphérique, comme représenté à la figure 2, la distance déterminée entre la face avant des éléments transducteurs du groupe donné au point de focalisation 10 est différente de la même distance par rapport au point de focalisation des autres groupes de fréquence de résonance différente, ce qui permet de réaliser une attaque électrique ou électronique de tous les groupes simultanément de manière que les pics de pression maximale coïncident.

Par exemple l'écart des distances de focalisation entre le premier groupe et le deuxième groupe est de l'ordre de 1/4 de longueur d'ondes.

On peut constater que cette compensation correspond à une compensation en temps ($\Delta t$) lorsque la distance focale est constante.

On peut ainsi observer que l'on peut soit mettre tous les éléments transducteurs à une distance focale identique du point de focalisation 10 avec une attaque électrique differente entre les groupes, tout en étant synchronisé pour obtenir des pics de pression maximale en coïncidence, ou bien on peut modifier la distance focale des divers groupes en réalisant alors une attaque électronique simultanée de tous les éléments transducteurs des différents groupes.

Une autre possibilité utilisable selon l'invention consiste à utiliser un moyen support plan comme décrit dans le document DE-A-31 19 295, la distance focale étant alors differente pour des éléments transducteurs qui peuvent être du même groupe et dans ce cas, on réalise une focalisation électronique comme cela résulte de la description précédente, ceci étant tout à fait clair à l'homme du métier de la technologie des ultrasons.

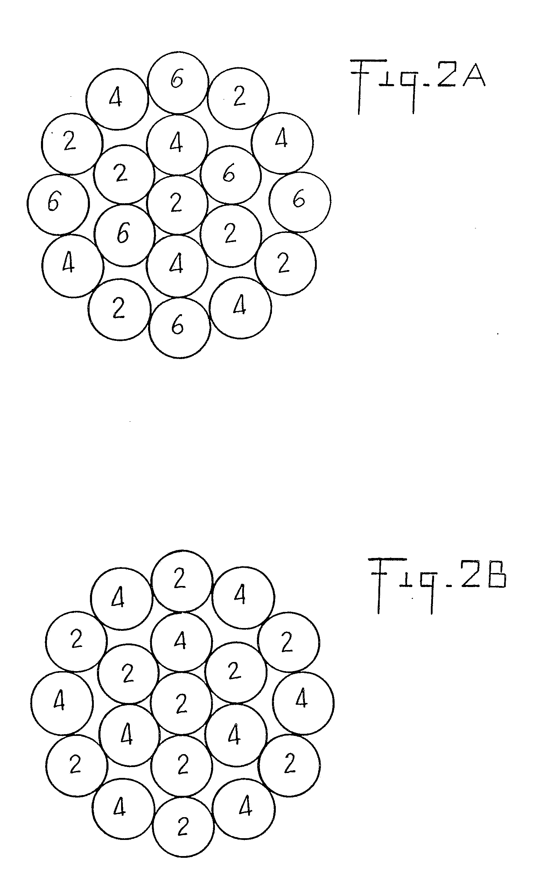

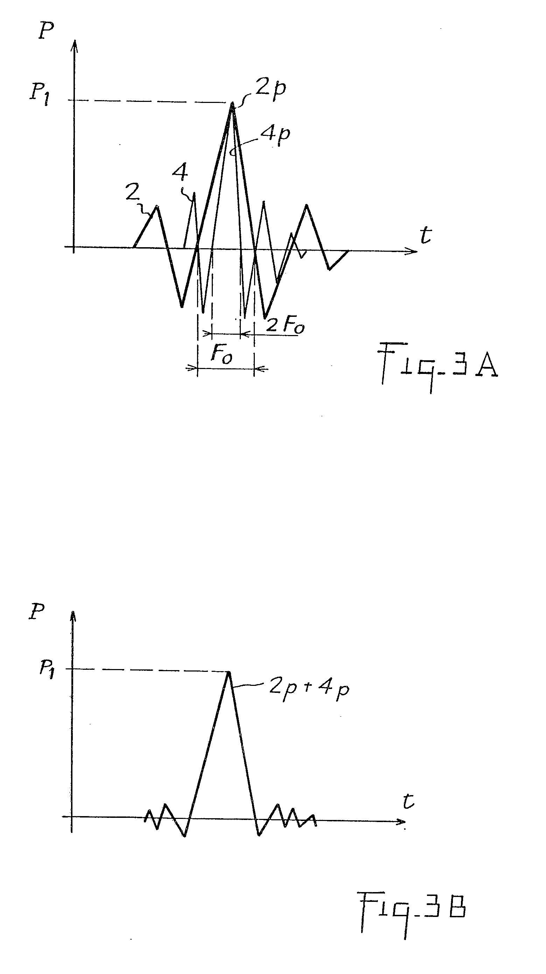

Grâce à cette combinaison d'éléments transducteurs de fréquence de résonance différente, on réduit de manière significative les ondes négatives émises tout en augmentant de manière significative le pic de pression de l'onde positive principale comme cela est illustré par les figures 3a et 3b par rapport aux figures 1a et 1b, et expliquées ci-après.

Pour simplifier, à la figure 3a, on a représenté schématiquement l'onde émise au cours du temps par le premier groupe d'éléments transducteurs 2, cette courbe étant également référencée 2, et par le deuxième groupe d'élémènts transducteurs 4, également référencé 4. On peut observer que l'onde positive principale 2p du premier groupe d'éléments transducteurs 2 et l'onde positive principale 4p émise par le deuxième groupe d'éléments transducteurs 4 ont leur sommet qui coïncident, et en conséquence vont s'ajouter pour donner une onde positive principale résultante, représentée à la figure 3b et repérée 2p + 4p.

Par contre, les ondes positives secondaires et les ondes négatives secondaires émises par chaque groupe des éléments transducteurs 2 ou des éléments transducteurs 4 vont s'annuler au moins partiellement, de sorte que l'on obtient la courbe d'onde résultante de la figure 3b.

On peut constater ainsi que l'on a réduit de manière significative l'onde négative. Cet effet sera amplifié par la présence du troisième groupe d'éléments transducteurs 6.

On aura ainsi réduit l'amplitude de l'onde négative à un niveau se situant en dessous du seuil pour lequel cette onde négative provoque un effet de cavitation capable de détruire ou d'endommager les cellules de tissu avoisinant la cible à détruire telle qu'une concrétion, ou un tissu malade tel qu'une tumeur, contrairement à ce qui était réalisé avec le dispositif générateur de l'art antérieur comprenant un groupe unique d'éléments transducteurs de fréquence de résonance unique.

En outre, et ceci constitue également un effet particulièrement inattendu et non évident pour un homme du métier, l'onde résultante obtenue selon l'invention a un pic de pression positive maximale qui est au moins égal au pic de pression antérieurement obtenu. On améliore ainsi l'efficacité de destruction de la cible présente au foyer par la présence en pratique d'un pic unique de pression, sans partie négative sensible.

On comprend ainsi que l'on obtient tous les avantages techniques déterminants précédemment énoncés.

Naturellement, la présente invention comprend tous les moyens constituant des équivalents techniques des moyens précédemment décrits.

Ainsi, les éléments transducteurs piézoélectriques peuvent être quelconques et par exemple à base de titanate-zirconate classique ou de toute constitution permettant de remplir la même fonction. De même, la dimension des éléments transducteurs individuels pourra varier dans de larges limites ainsi que le rayon de la coupole sphérique. Egalement, il n'est pas obligatoire que les éléments transducteurs soient disposés sur une coupole sphérique car la focalisation peut être réalisée électroniquement comme enseigné par exemple par le document DE-A-3 119 295, en agissant sur les moments d'attaque électrique de chaque groupe d'éléments transducteurs.

**Revendications**

1. Dispositif générateur d'ondes ultrasoniques focalisées en un foyer, à ondes négatives réduites, comprenant une pluralité d'éléments transducteurs (2, 4, 6) de type piézoélectrique disposés extérieurement sur un moyen support de focalisation en un point de focalisation ou foyer (10), par exemple une coupole de forme sphérique, caractérisé en ce que lesdits éléments transducteurs (2, 4, 6) sont répartis en au moins deux groupes d'éléments transducteurs dont la fréquence de résonance diffère de telle sorte que les ondes ultrasonique positives principales émises par chaque groupe s'ajoutent et les ondes ultrasoniques secondaires positives et négatives s'annulent au moins partiellement.

2. Dispositif selon la revendication 1, caractérisé en ce que le deuxième groupe d'éléments transducteurs (4), ou les autres groupes d'éléments transducteurs, a une fréquence de résonance $F_o$ voisine ou égale à un multiple ou sous multiple de la fréquence de résonance des éléments transducteurs du premier groupe (2).

3. Dispositif selon la revendication 1 ou 2, caractérisé en ce que la fréquence de résonance $F_o$ du deuxième groupe d'éléments transducteurs (4) est égale ou voisine du double de la fréquence de résonance du premier groupe d'éléments transducteurs (2).

4. Dispositif selon l'une quelconque des revendications 1 à 3, caractérisé en ce que les éléments transducteurs de chaque groupe sont disposés alternativement de telle sorte que la surface totale occupée par les éléments de chaque groupe soit sensiblement identique.

5. Dispositif selon l'une quelconque des revendications 1 à 4 pour lequel le moyen support est constitué par une coupole sphérique, caractérisé en ce que tous les éléments transducteurs (2, 4, 6) des divers groupes sont à la même distance du point de focalisation (10), dite distance focale, qui est de préférence égale au rayon de courbure de la coupole, l'attaque électronique de chaque groupe de fréquence donnée est réalisée de façon retardée ou avancée en fonction du groupe attaqué par rapport au groupe de référence, l'écart de temps (retard ou avance) étant donné par la formule suivante :

$$\Delta t = \lambda / 4V$$

dans laquelle :
$\Delta$ est la vitesse ultrasonore dans le milieu remplissant la coupole, en général constitué par de l'eau,
$\lambda$ est la longeur d'ondes du groupe attaqué.

6. Dispositif selon l'une quelconque des revendications 1 à 4, pour lequel le moyen support est constitué par une coupole sphérique, caractérisé en ce que la distance déterminée entre la face avant des éléments transducteurs d'un groupe donné et le point de focalisation est différente de la même distance au point de focalisation des autres groupes de fréquence de résonance différente, l'attaque électrique de tous les groupes étant simultanée, pour que les pics de pression maximale coïncident.

7. Dispositif selon l'une quelconque des revendications 1 à 6, caractérisé en ce qu'il comprend un troisième groupe d'éléments transducteurs (6) dont la fréquence de résonance est égale ou voisine de 4 fois la fréquence de résonance $F_o$ du premier groupe d'éléments transducteurs (2).

8. Dispositif selon l'une quelconque des revendications 1 à 7, caractérisé en ce que la fréquence de résonance des éléments transducteurs (2) du premier groupe est égale à 0,5 mégaHertz.

9. Utilisation du dispositif tel que défini selon l'une quelconque des revendications 1 à 8, pour la lithotritie extra corporelle ou pour la destruction de tissus particuliers.

Fig-1A

Fig-1B

Fig-2

Fig.2A

Fig.2B

Fig-3A

Fig-3B

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A,D | FR-A-2 567 394 (RICHARD WOLF GmbH) * Résumé; figure 1* & DE-A-34 25 992 *<br>--- | 1 | G 10 K 11/34 |
| A | FR-A-2 579 355 (RICHARD WOLF GmbH) * Résumé; figure 1 *<br>--- | 1 | |
| A | US-A-3 656 012 (DIXON) * Résumé *<br>--- | 1 | |
| A | US-A-3 879 698 (PEPPER) * Résumé *<br>--- | 1 | |
| A | FR-A-2 571 635 (RICHARD WOLF GmbH) * Résumé; figures 1,2 *<br>--- | 1 | |
| E,A | EP-A-0 289 382 (EDAP INTERNATIONAL) * Colonne 4, ligne 54 - colonne 5, ligne 49; figures 1-4 *<br>----- | 1-5,7-9 | |

| | DOMAINES TECHNIQUES RECHERCHES (Int. Cl.4) |
|---|---|
| | G 10 K<br>A 61 B |

**Le présent rapport a été établi pour toutes les revendications**

| Lieu de la recherche | Date d'achèvement de la recherche | Examinateur |
|---|---|---|
| LA HAYE | 12-12-1988 | ANDERSON A.TH. |

EPO FORM 1503 03.82 (P0402)